# EUROPÄISCHE PATENTSCHRIFT

(11) **EP 2 618 800 B1**
(45) Veröffentlichungstag und Bekanntmachung des Hinweises auf die Patenterteilung: **26.04.2017**
(21) Anmeldenummer: 11752187.2
(22) Anmeldetag: 31.08.2011
(51) Int. Cl.: A61Q 5/06, A61K 8/73, A61K 8/81, A61K 8/04

(54) **MITTEL ZUR TEMPORÄREN VERFORMUNG KERATINHALTIGER FASERN**
COMPOSITION FOR TEMPORARY SHAPING OF KERATINOUS FIBERS
AGENT DE DÉFORMATION TEMPORAIRE DE FIBRES À TENEUR EN KÉRATINE

(30) Priorität: 23.09.2010 DE 102010041267
(43) Veröffentlichungstag der Anmeldung: 31.07.2013
(73) Patentinhaber: Henkel AG & Co. KGaA, 40589 Düsseldorf (DE)
(72) Erfinder: MÜLLER, Burkhard, 21075 Hamburg (DE); KAFTAN, Pamela, 22525 Hamburg (DE)
(86) Internationale Anmeldenummer: PCT/EP2011/064963
(87) Internationale Veröffentlichungsnummer: WO 2012/038205

(56) Entgegenhaltungen:
- WO-A1-2005/060926
- DE-A1- 10 358 587
- DE-A1-102008 030 661
- US-A1- 2006 084 586
- US-A1- 2008 102 051
- US-A1- 2010 069 601
- JONATHAN MOORE ET AL: "Modification of polymer properties using a non-ionic surfactant", RESEARCH DISCLOSURE, MASON PUBLICATIONS, HAMPSHIRE, GB, Bd. 441, Nr. 121, 1. Januar 2001 (2001-01-01), XP007127510, ISSN: 0374-4353

## Beschreibung

Die vorliegende Erfindung betrifft Mittel zur temporären Verformung keratinhaltiger Fasern, enthaltend in einem kosmetischen Träger eine Kombination aus mindestens einer kationischen Celluloseverbindung, mindestens einem Copolymer umfassend Pyrrolidon, Caprolactam und Dialkylaminoethyl-Reste und mindestens einem davon verschiedenen Copolymer umfassend Pyrrolidon und Dialkylaminoalkyl-Reste, die Verwendung dieser Mittel zur temporären Verformung und/oder zur Pflege keratinhaltiger Fasern und Aerosolhaarsprays/-schäume auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für die Kombination von starkem Halt einerseits und einfacher, gleichmäßiger Applikation auf die keratinhaltigen Fasern andererseits.

Aufgabe der vorliegenden Erfindung war es daher, ein Mittel zur temporären Verformung keratinhaltiger Fasern zur Verfügung zu stellen, das sich durch einen hohen Haltegrad auszeichnet. Weiterhin soll das Mittel eine gute Volumenwirkung sowie eine hohe Pflegeleistung zeigen. Die Bildung von Filmplaken - insbesondere die Bildung entsprechender Rückstände bei der Anwendung, dem Ausbürsten und der Trocknung der keratinhaltigen Faser - soll vermieden werden.

Es wurde nunmehr überraschenderweise gefunden, dass dies durch eine Kombination von mindestens einer kationischen Celluloseverbindung mit mindestens einem Copolymer umfassend Pyrrolidon-N-yl, Caprolactam-N-yl und Dialkylaminoethyl-Reste und mindestens einem davon verschiedenen Copolymer umfassend Pyrrolidon-N-yl und Dialkylaminoalkyl-Reste erreicht werden kann. Die mit dieser Kombination erzielten Zusammensetzungen weisen sogar - wenn sie als Haarschaum konfektioniert werden - besonders gute Schaumparameter auf.

Ein erster Gegenstand der vorliegenden Erfindung ist daher ein Mittel zur Behandlung keratinhaltiger Fasern, insbesondere menschlichem Haar, enthaltend in einem kosmetischen Träger
(a) mindestens eine kationische Celluloseverbindung
   und
(b) mindestens ein Copolymer umfassend mindestens eine Struktureinheit der Formel (I) und mindestens eine Struktureinheit der Formel (II) und mindestens eine Struktureinheit der Formel (III) 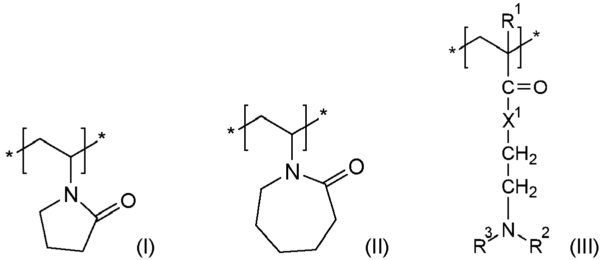 worin
   R¹ ein Wasserstoffatom oder eine Methylgruppe bedeutet,
   X¹ ein Sauerstoffatom oder eine Gruppe NH bedeutet,
   R² und R³ unabhängig voneinander für eine (C₁ bis C₄)-Alkylgruppe stehen
   und
(c) mindestens ein Copolymer umfassend mindestens eine Struktureinheit der Formel (I) und mindestens eine Struktureinheit der Formel (IV) 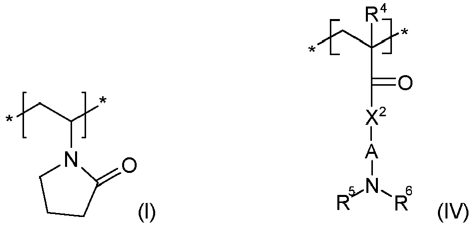 worin
   R⁴ ein Wasserstoffatom oder eine Methylgruppe bedeutet,
   X² ein Sauerstoffatom oder eine Gruppe NH bedeutet,
   A eine (C₂ bis C₃)-Alkylengruppe (insbesondere Ethan-1,2-diyl oder Propan-1,3-diyl),
   R⁵ und R⁶ unabhängig voneinander für eine (C₁ bis C₄)-Alkylgruppe stehen.

Die in dem erfindungsgemäßen Mittel enthaltenen Verbindungen der Komponente (a), (b) und (c) sind voneinander verschieden.

Gemäß obiger Formeln und allen folgenden Formeln steht eine chemische Bindung, die mit dem Symbol * gekennzeichnet ist, für eine freie Valenz des entsprechenden Strukturfragments.

Es erweisen sich bevorzugt solche kationischen Celluloseverbindungen als im Sinne der Erfindung vorteilhaft, die in mindestens einer Seitenkette mehr als eine permanente kationische Ladung tragen. Cellulose ist aus β-1,4-glycosidisch-verknüpften D-Glucopyranose-Einheiten aufgebaut und bildet unverzweigte, wasserunlösliche Ketten. Als "Seitenkette" einer Cellulose werden chemische Substituenten definiert, die an das Celluloserückrat binden und nicht zur nativen Cellulose zählen, da sie z.B. durch chemische Synthese nachträglich eingeführt wurden.

Ferner sind unter den kationischen Celluloseverbindungen solche hervorzuheben, die aus Reaktion von Hydroxy(C₂ bis C₄)alkylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Die kationischen Celluloseverbindungen (a) sind in den erfindungsgemäßen Mitteln bevorzugt in einer Menge von 0,1 Gew.-% bis 3,0 Gew.-%, besonders bevorzugt von 0,4 Gew.-% bis 1,8 Gew.-%, ganz besonders bevorzugt von 0,8 Gew.-% bis 1,2 Gew.-%, jeweils bezogen auf das Gewicht des erfindungsgemäßen Mittels, enthalten.

Erfindungsgemäß bevorzugte Mittel enthalten mindestens solch ein Copolymer (b), das gemäß Formel (III) mindestens einen der folgenden Parameter (besonders bevorzugt alle drei gemeinsam) erfüllt:
- R¹ steht für eine Methylgruppe,
- X¹ steht für ein Sauerstoffatom,
- R² und R³ stehen für eine Methylgruppe.

Ganz besonders bevorzugt enthält das erfindungsgemäße Mittel als Copolymer (b) ein Terpolymer das erhalten wird aus N-Vinylpyrrolidon, N-Vinylcaprolactam und N,N-Dimethylaminoethylmethacrylat. Solche Copolymere können beispielsweise unter dem Handelsnamen Advantage S (INCI-Bezeichnung: Vinylcaprolactam/VP/Dimethylaminoethylmethacrylate Copolymer, in Pulverform), Advantage LC-E (INCI-Bezeichnung: Vinylcaprolactam/VP/Dimethylaminoethylmethacrylate Copolymer, Laurylpyrrolidone; 37 Gew.-% Aktivsubstanz in Ethanol mit Zusatz von N-Laurylpyrrolidon) oder Advantage LC-A (INCI-Bezeichnung: Vinylcaprolactam/VP/Dimethylaminoethylmethacrylate Copolymer; 37 Gew.-% Aktivsubstanz in Ethanol) von der Firma ISP bezogen werden.

Die Copolymere (b) sind in den erfindungsgemäßen Mitteln bevorzugt in einer Menge von 0,1 Gew.-% bis 10,0 Gew.-%, besonders bevorzugt von 0,5 Gew.-% bis 8,0 Gew.-%, ganz besonders bevorzugt von 0,5 Gew.-% bis 5,0 Gew.-%, jeweils bezogen auf das Gewicht des erfindungsgemäßen Mittels, enthalten.

Im Rahmen einer weiteren bevorzugten Ausführungsform enthält das erfindungsgemäße Mittel solch ein Copolymer (c), das gemäß Formel (IV) mindestens einen der folgenden Parameter (besonders bevorzugt alle vier gemeinsam) erfüllt:
- R⁴ steht für eine Methylgruppe,
- X² steht für ein Sauerstoffatom,
- A steht für eine Ethan-1,2-diyl-Gruppe,
- R⁵ und R⁶ stehen für eine Methylgruppe.

Falls der Rest X² gemäß Formel (IV) für ein Sauerstoffatom steht ist es wiederum bevorzugt, wenn das Copolymer (c) zusätzlich mindestens eine Struktureinheit der Formel (V) umfasst 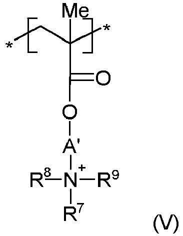 in der
R⁸ und R⁹ unabhängig voneinander für eine (C₁ bis C₆)-Alkylgruppe (insbesondere für Methyl) stehen, A' für Ethan-1,2-diyl oder Ethan-1,3-diyl (insbesondere Ethan-1,2-diyl) und R⁷ für eine (C₁ bis C₄)-Alkylgruppe (insbesondere für Ethyl) steht.

Zur Kompensation der positiven Polymerladung der Struktureinheit der Formel (V) in dem erfindungsgemäßen Mittel (und falls nicht anders beschrieben auch für alle weiteren kationischen Verbindungen des erfindungsgemäßen Mittels) dient mindestens ein physiologisch verträgliches Anion, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugt enthält das erfindungsgemäße Mittel dieser Ausführungsform als Copolymer (c) ein Copolymer das erhalten wird aus N-Vinylpyrrolidon und N,N-Dimethylaminoethylmethacrylat, welches mit Diethylsulfat kationisiert wurde. Solche Copolymere fallen unter die INCI-Bezeichnung Polyquaternium-11. Sie können beispielsweise unter dem Handelsnamen Gafquat 755 oder Gafquat 734 von der Firma ISP oder unter dem Handelsnamen Luviquat PQ 11 PN von der Firma BASF SE bezogen werden.

Im Rahmen einer weiteren bevorzugten Ausführungsform enthält das erfindungsgemäße Mittel solch ein Copolymer (c), das gemäß Formel (IV) mindestens einen der folgenden Parameter (besonders bevorzugt alle vier gemeinsam) erfüllt:
- R⁴ steht für eine Methylgruppe,
- X² steht für eine Gruppe NH,
- A steht für eine Propan-1,3-diyl-Gruppe,
- R⁵ und R⁶ stehen für eine Methylgruppe.

Ganz besonders bevorzugt enthält das erfindungsgemäße Mittel dieser Ausführungsform als Copolymer (c) ein Copolymer, das erhalten wird aus N-Vinylpyrrolidon und N,N-Dimethylaminopropylmethacrylamid. Solche Copolymere fallen unter die INCI-Bezeichnung VP/DMAPA Acrylates Copolymer. Sie können beispielsweise unter dem Handelsnamen Styleze CC 10 (ca. 10 Gew.-% Polymerlösung in Wasser) von der Firma ISP bezogen werden.

Folglich ist es erfindungsgemäß besonders bevorzugt, wenn gemäß Formel (IV)
R⁴ eine Methylgruppe bedeutet,
X² ein Sauerstoffatom oder eine Gruppe NH bedeutet,
A für Ethan-1,2-diyl oder Propan-1,3-diyl steht und
R⁵ und R⁶ unabhängig voneinander für eine (C₁ bis C₄)-Alkylgruppe (insbesondere Methyl) stehen, mit der Maßgabe, dass
wenn X² für ein Sauerstoffatom steht, A Ethan-1,2-diyl bedeutet,
wenn X² für eine Gruppe NH steht, A Propan-1,3-diyl bedeutet.

Die Copolymere (c) sind in den erfindungsgemäßen Mitteln bevorzugt in einer Menge von 0,01 Gew.-% bis 5,0 Gew.-%, besonders bevorzugt von 0,1 Gew.-% bis 2,0 Gew.-%, ganz besonders bevorzugt von 0,15 Gew.-% bis 1,5 Gew.-%, jeweils bezogen auf das Gewicht des erfindungsgemäßen Mittels, enthalten.

Erfindungsgemäß besonders bevorzugte Mittel enthalten in einem kosmetischen Träger
(a) mindestens eine kationische Celluloseverbindung, die aus Reaktion von Hydroxy(C₂ bis C₄)alkylcellulose mit einem Dimethyldiallylammonium-Reaktanden (insbesondere Dimethyldiallylammoniumchlorid) gegebenenfalls in Gegenwart weiterer Reaktanden hergestellt werden,
   und
(b) mindestens ein Copolymer umfassend mindestens eine Struktureinheit der Formel (I) und mindestens eine Struktureinheit der Formel (II) und mindestens eine Struktureinheit der Formel (III) 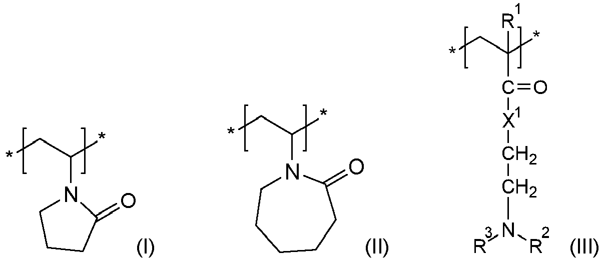 worin
   R¹ eine Methylgruppe bedeutet, X¹ ein Sauerstoffatom bedeutet und R² und R³ für eine Methylgruppe stehen
   und
(c) mindestens ein Copolymer umfassend mindestens eine Struktureinheit der Formel (I) und mindestens eine Struktureinheit der Formel (IV) 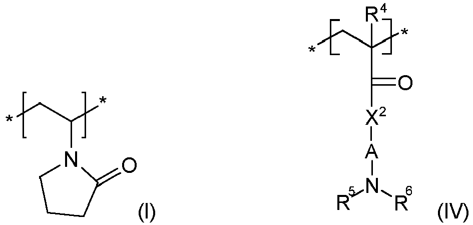 worin
   R⁴ ein Wasserstoffatom oder eine Methylgruppe bedeutet,
   X² ein Sauerstoffatom oder eine Gruppe NH bedeutet,
   A eine (C₂ bis C₃)-Alkylengruppe (insbesondere Ethan-1,2-diyl oder Propan-1,3-diyl),
   R⁵ und R⁶ unabhängig voneinander für eine (C₁ bis C₄)-Alkylgruppe stehen.

Erfindungsgemäß ganz besonders bevorzugte Mittel enthalten in einem kosmetischen Träger
(a) mindestens eine kationische Celluloseverbindung, die aus Reaktion von Hydroxy(C₂ bis C₄)alkylcellulose mit einem Dimethyldiallylammonium-Reaktanden (insbesondere Dimethyldiallylammoniumchlorid) gegebenenfalls in Gegenwart weiterer Reaktanden hergestellt werden,
   und
(b) mindestens ein Copolymer umfassend mindestens eine Struktureinheit der Formel (I) und mindestens eine Struktureinheit der Formel (II) und mindestens eine Struktureinheit der Formel (III) 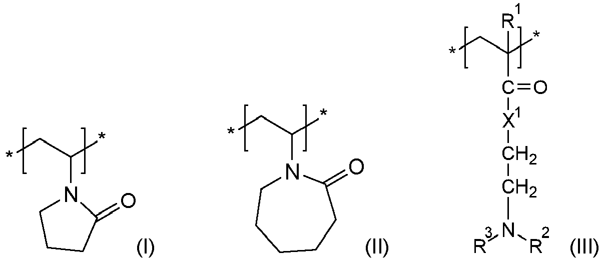 worin
   R¹ eine Methylgruppe bedeutet, X¹ ein Sauerstoffatom bedeutet und R² und R³ für eine Methylgruppe stehen
   und
(c) mindestens ein Copolymer umfassend mindestens eine Struktureinheit der Formel (I) und mindestens eine Struktureinheit ausgewählt aus Formel (IV) 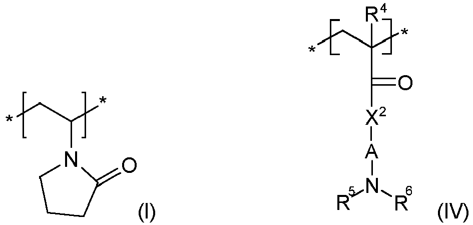 worin
   R⁴ eine Methylgruppe bedeutet,
   X² ein Sauerstoffatom oder eine Gruppe NH bedeutet,
   A für Ethan-1,2-diyl oder Propan-1,3-diyl steht und
   R⁵ und R⁶ unabhängig voneinander für eine (C₁ bis C₄)-Alkylgruppe stehen,
      mit der Maßgabe, dass
      wenn X² für ein Sauerstoffatom steht, bedeutet A Ethan-1,2-diyl,
      wenn X² für eine Gruppe NH steht, bedeutet A Propan-1,3-diyl.

Am bevorzugtesten eignen sich erfindungsgemäße Mittel, die in einem kosmetischen Träger enthalten
(a) mindestens eine kationische Celluloseverbindung der INCI-Bezeichnung Polyquaternium-4 und
(b) mindestens ein Copolymer der INCI-Bezeichnung Vinylcaprolactam/VP/Dimethylaminoethyl-methacrylate Copolymer
   und
(c) mindestens ein Copolymer ausgewählt aus mindestens einem Copolymer der Gruppe, die gebildet wird aus Coplymeren der INCI-Bezeichnung Polyquaternium-11 und Copolymeren der INCI-Bezeichnung VP/DMAPA Acrylates Copolymer.

Die INCI (INCI = International Nomenclature of Cosmetic Ingredients, i.e. Internationale Nomenklatur für kosmetische Inhaltsstoffe) bezeichnet eine internationale Richtlinie für die korrekte und einheitliche Angabe der Inhaltsstoffe für Kosmetika. Eine der INCI entnommene Bezeichnung eines kosmetischen Inhaltsstoffes bzw. einer Gruppe kosmetischer Inhaltsstoffe wird erfindungsgemäß als INCI-Bezeichnung definiert.

Folgende Ausführungsformen (A) bis (D) sind erfindungsgemäß bevorzugte Ausführungsformen:
(A): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen
      und
   (b) 0,1 bis 10 Gew.-% eines oder mehrerer Copolymere umfassend mindestens eine Struktureinheit der Formel (I) und mindestens eine Struktureinheit der Formel (II) und mindestens eine Struktureinheit der Formel (III) 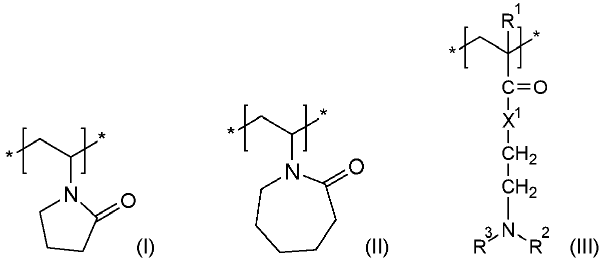 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,01 bis 5,0 Gew.-% eines oder mehrerer Copolymere umfassend mindestens eine Struktureinheit der Formel (I) und mindestens eine Struktureinheit der Formel (IV) 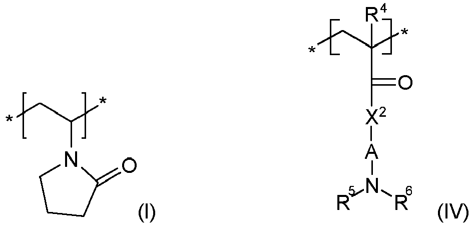 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen.
(B): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen
      und
   (b) 0,5 bis 5,0 Gew.-% eines oder mehrerer Copolymere umfassend mindestens eine Struktureinheit der Formel (I) und mindestens eine Struktureinheit der Formel (II) und mindestens eine Struktureinheit der Formel (III) 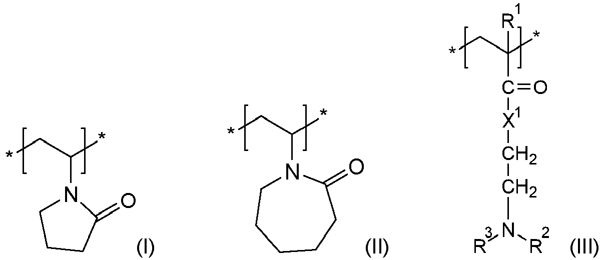 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,15 bis 1,5 Gew.-% eines oder mehrerer Copolymere umfassend mindestens eine Struktureinheit der Formel (I) und mindestens eine Struktureinheit der Formel (IV) 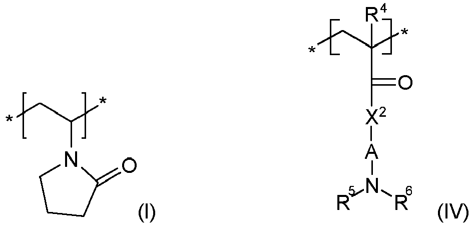 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen.
(C): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen der INCI-Bezeichnung Polyquaternium-4
      und
   (b) 0,1 bis 10,0 Gew.-% eines oder mehrerer Copolymere der INCI-Bezeichnung Vinylcaprolactam/VP/Dimethylaminoethyl-methacrylate Copolymer
      und
   (c) 0,01 bis 5,0 Gew.-% eines oder mehrerer Copolymere ausgewählt aus mindestens einem Copolymer der Gruppe, die gebildet wird aus Coplymeren der INCI-Bezeichnung Polyquaternium-11 und Copolymeren der INCI-Bezeichnung VP/DMAPA Acrylates Copolymer.
(D): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen der INCI-Bezeichnung Polyquaternium-4
      und
   (b) 0,5 bis 5,0 Gew.-% eines oder mehrerer Copolymere der INCI-Bezeichnung Vinyl-caprolactam/VP/Dimethylaminoethylmethacrylate Copolymer
      und
   (c) 0,15 bis 1,5 Gew.-% eines oder mehrerer Copolymere ausgewählt aus mindestens einem Copolymer der Gruppe, die gebildet wird aus Coplymeren der INCI-Bezeichnung Polyquaternium-11 und Copolymeren der INCI-Bezeichnung VP/DMAPA Acrylates Copolymer.

Die erfindungsgemäßen Mittel enthalten die Inhalts- bzw. Wirkstoffe in einem kosmetischen Träger. Bevorzugte kosmetischen Träger sind wässrige, alkoholische oder wässrigalkoholische Medien. Das erfindungsgemäße Mittel enthält vorzugsweise mindestens 10 Gew.-% Wasser, besonders bevorzugt mindestens 40 Gew.-% Wasser, ganz besonders bevorzugt mindestens 60 Gew.-% Wasser, jeweils bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Es ist im Rahmen einer speziellen Ausführungsform der Erfindung möglich, mindestens einen (C₁ bis C₄)-Monoalkylalkohol in den erfindungsgemäßen Mitteln insbesondere in einer Menge von 1 bis 50 Gew.-% insbesondere von 5 bis 20 Gew.-% einzusetzen. Dies ist wiederum insbesondere für die Konfektionierung als Pumpschaum, Pumpspray oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe mit mehr als 5 Kohlenstoffatomen, wie Hexan, Isopentan und cyclische Kohlenwasserstoffe wie Cyclohexan. Weitere besonders bevorzugte wasserlösliche Lösungsmittel sind Glycerin, Ethylenglykol und Propylenglykol bevorzugt in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Neben dem besagten Polymeren (a), (b) und (c) enthält das erfindungsgemäße Mittel bevorzugt zusätzlich mindestens ein kationisches Tensid.

Die kationischen Tenside sind in dem erfindungsgemäßen Mittel bevorzugt in einer Menge von 0,01 bis 2,0 Gew.-%, insbesondere von 0,1 bis 0,5 Gew.-%, enthalten.

Erfindungsgemäß einsetzbar sind bevorzugt kationische Tenside vom Typ der quartären Ammoniumverbindungen (wie (C₈ bis C₂₂)-Alkyltrimethylammoniumverbindungen, Di(C₈ bis C₂₂)alkyldimethylammoniumverbindungen, Tri(C₈ bis C₂₂)alkylmethylammoniumverbindungen), (C₈ bis C₂₂)Alkyltri(oligoalkoxy)ammoniumverbindungen, Di(C₈ bis C₂₂)Alkyldi(oligoalkoxy)ammoniumverbindungen der Esterquats und der Amidoamine. Besonders bevorzugte kationische Tenside werden ausgewählt unter den N-(C₁₂ bis C₂₀)AlkylN,N,N-trimethylammonium-Verbindungen, insbesondere deren Chloriden oder Bromiden, wie insbesondere unter Cetyltrimethylammoniumchlorid, Cetyltrimethylammoniumbromid, Stearyltrimethylammonium-chlorid, Stearyltrimethylammoniumbromid.

Ein weiteres bevorzugt einsetzbares kationisches Tensid besitzt die allgemeine Formel (VI), 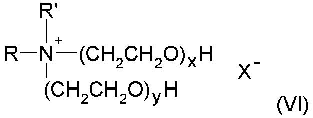 worin
x und y stehen unabhängig voneinander für eine ganze Zahl größer 0,
R steht für eine (C₈ bis C₂₀)-Alkylgruppe oder eine (C₈ bis C₂₀)-Alkenylgruppe,
R' steht für eine Gruppe *-(CH₂CH₂O)_{z}H worin z eine ganze Zahl größer 0 bedeutet, eine (C₈ bis C₂₀)-Alkylgruppe oder eine (C₈ bis C₂₀)-Alkenylgruppe,
X⁻ steht für ein physiologisch verträgliches Anion.

Besagtes kationisches Tensid mit der Formel (VI) ist in den erfindungsgemäßen Mitteln bevorzugt in einer Menge von 0,1 Gew.-% bis 2 Gew.-%, bezogen auf das Gewicht des erfindungsgemäßen Mittels, enthalten.

Dabei ist es erfindungsgemäß bevorzugt, wenn das kationische Tensid der Formel (VI) ein Molekulargewicht von 550 bis 2500 g/mol, besonders bevorzugt von 600 bis 1000 g/mol, aufweist.

Der Rest R gemäß Formel (VI) steht bevorzugt für Dodecyl, Tetradecyl, Hexadecyl oder Octadecyl.

Es ist erfindungsgemäß bevorzugt, wenn R' gemäß Formel (VI) für eine Gruppe *-(CH₂CH₂O)_{z}H steht, worin z eine ganze Zahl größer 0 bedeutet.

X-steht gemäß Formel (VI) bevorzugt für Chlorid, Phosphat oder Dihydrogenphosphat.

Darüber hinaus stellte es sich als bevorzugt heraus, wenn gemäß Formel (IV) x und y (und falls R' für die Gruppe *-(CH₂CH₂O)_{z}H steht auch z) unabhängig voneinander ausgewählt werden unter einer ganzen Zahl ausgewählt aus 1, 2, 3, 4, 5, 6, 7, 8, 9, 10.

Ferner ist es erfindungsgemäß bevorzugt, wenn die Summe aus x und y gemäß Formel (VI) (bzw. wenn R' für eine Gruppe *-(CH₂CH₂O)_{z}H steht, die Summe aus x und y und z,) für eine Zahl zwischen 3 und 20, insbesondere für eine Zahl zwischen 5 und 15 steht.

Ein besonders bevorzugtes kationisches Tensid weist die Formel (VIa) auf 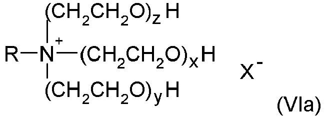 worin
x und y und z stehen unabhängig voneinander für eine ganze Zahl größer 0 und die Summe aus (x + y + z) steht für eine Zahl zwischen 3 und 20, insbesondere zwischen 5 und 15,
R steht für eine (C₈ bis C₂₀)-Alkylgruppe oder eine (C₈ bis C₂₀)-Alkenylgruppe,
X⁻ steht für ein physiologisch verträgliches Anion.

Ein ganz besonders bevorzugtes kationisches Tensid ist das Tris(oligooxyethyl)alkylammonium-Dihydrogenphosphat-Salz mit dem Molekulargewicht von 780 g/mol, das mit der INCI-Bezeichnung Quaternium-52 beispielsweise unter dem Handelsnamen Dehyquart^{®} SP von der Firma Cognis vertrieben wird.

Darüber hinaus ist es erfindungsgemäß besonders bevorzugt, wenn die erfindungsgemäßen Mittel als kationisches Tensid neben mindestens einer N-(C₁₂ bis C₂₂)Alkyl-N,N,N-trimethylammonium-Verbindung zusätzlich mindestens eine Verbindung gemäß obiger Formel (VI) (insbesondere obiger Formel (VIa) enthält.

Folgende erfindungsgemäße Mittel (E) bis (L) gelten im Sinne der Erfindung als besonders bevorzugt:
(E): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen
      und
   (b) 0,1 bis 10 Gew.-% eines oder mehrerer Copolymere umfassend mindestens eine Struktureinheit der Formel (I) und mindestens eine Struktureinheit der Formel (II) und mindestens eine Struktureinheit der Formel (III) 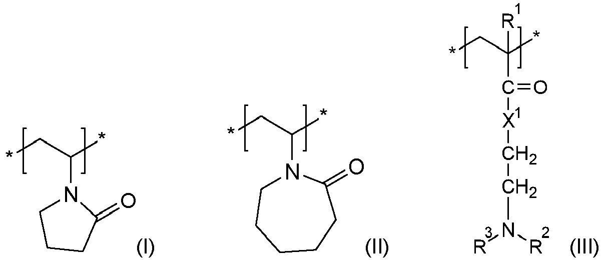 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,01 bis 5,0 Gew.-% eines oder mehrerer Copolymere umfassend mindestens eine Struktureinheit der Formel (I) und mindestens eine Struktureinheit der Formel (IV) 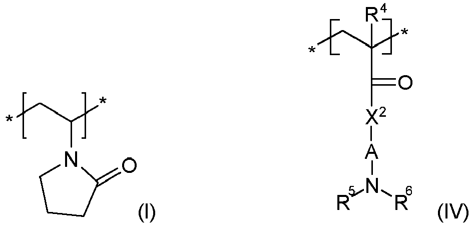 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen,
      und
   (d) mindestens ein kationisches Tensid, ausgewählt unter N-(C₁₂ bis C₂₂)Alkyl-N,N,N-trimethylammonium-Verbindungen.
(F): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen
      und
   (b) 0,5 bis 5,0 Gew.-% eines oder mehrerer Copolymere umfassend mindestens eine Struktureinheit der Formel (I) und mindestens eine Struktureinheit der Formel (II) und mindestens eine Struktureinheit der Formel (III) 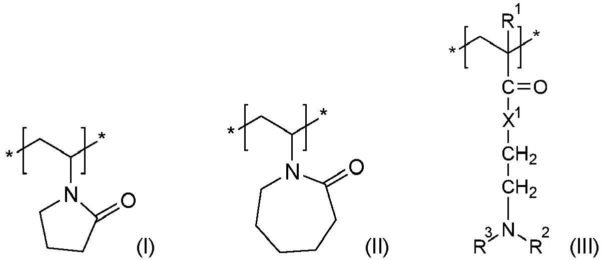 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,15 bis 1,5 Gew.-% eines oder mehrerer Copolymere umfassend mindestens eine Struktureinheit der Formel (I) und mindestens eine Struktureinheit der Formel (IV) 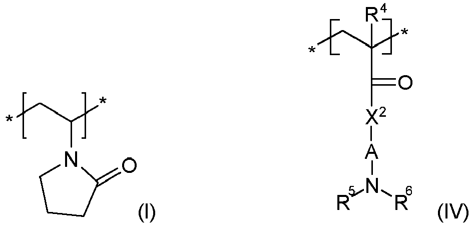 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen
      und
   (d) mindestens ein kationisches Tensid, ausgewählt unter N-(C₁₂ bis C₂₂)Alkyl-N,N,N-trimethylammonium-Verbindungen.
(G): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen der INCI-Bezeichnung Polyquaternium-4
      und
   (b) 0,1 bis 10,0 Gew.-% eines oder mehrerer Copolymere der INCI-Bezeichnung Vinylcaprolactam/VP/Dimethylaminoethyl-methacrylate Copolymer
      und
   (c) 0,01 bis 5,0 Gew.-% eines oder mehrerer Copolymere ausgewählt aus mindestens einem Copolymer der Gruppe, die gebildet wird aus Copolymeren der INCI-Bezeichnung Polyquaternium-11 und Copolymeren der INCI-Bezeichnung VP/DMAPA Acrylates Copolymer
      und
   (d) mindestens ein kationisches Tensid, ausgewählt unter N-(C₁₂ bis C₂₂)Alkyl-N,N,N-trimethylammonium-Verbindungen.
(H): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen der INCI-Bezeichnung Polyquaternium-4
      und
   (b) 0,5 bis 5,0 Gew.-% eines oder mehrerer Copolymere der INCI-Bezeichnung Vinylcaprolactam/VP/Dimethylaminoethylmethacrylate Copolymer
      und
   (c) 0,15 bis 1,5 Gew.-% eines oder mehrerer Copolymere ausgewählt aus mindestens einem Copolymer der Gruppe, die gebildet wird aus Copolymeren der INCI-Bezeichnung Polyquaternium-11 und Copolymeren der INCI-Bezeichnung VP/DMAPA Acrylates Copolymer
      und
   (d) mindestens ein kationisches Tensid, ausgewählt unter N-(C₁₂ bis C₂₂)Alkyl-N,N,N-trimethylammonium-Verbindungen.
(I): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen
      und
   (b) 0,1 bis 10 Gew.-% eines oder mehrerer Copolymere umfassend mindestens eine Struktureinheit der Formel (I) und mindestens eine Struktureinheit der Formel (II) und mindestens eine Struktureinheit der Formel (III) 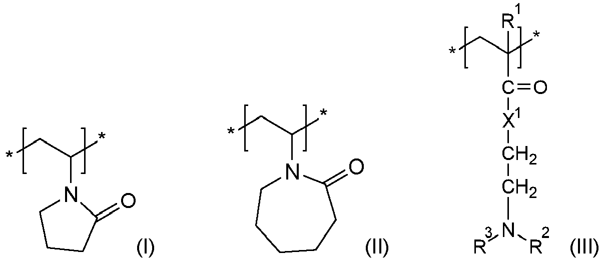 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,01 bis 5,0 Gew.-% eines oder mehrerer Copolymere umfassend mindestens eine Struktureinheit der Formel (I) und mindestens eine Struktureinheit der Formel (IV) 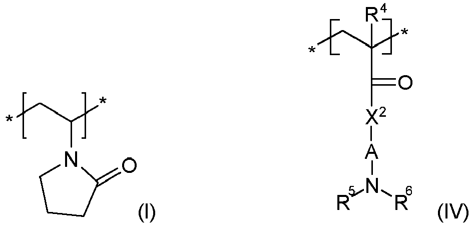 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen,
      und
   (d) mindestens ein kationisches Tensid, ausgewählt unter N-(C₁₂ bis C₂₂)Alkyl-N,N,N-trimethylammonium-Verbindungen
      und
   (e) mindestens ein kationisches Tensid gemäß Formel (VI), 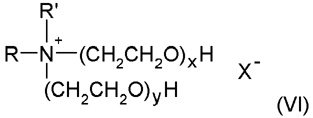 worin
      x und y stehen unabhängig voneinander für eine ganze Zahl größer 0,
      R steht für eine (C₈ bis C₂₀)-Alkylgruppe oder eine (C₈ bis C₂₀)-Alkenylgruppe,
      R' steht für eine Gruppe *-(CH₂CH₂O)_{z}H worin z eine ganze Zahl größer 0 bedeutet, eine (C₈ bis C₂₀)-Alkylgruppe oder eine (C₈ bis C₂₀)-Alkenylgruppe,
      X⁻ steht für ein physiologisch verträgliches Anion.
(J): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen
      und
   (b) 0,5 bis 5,0 Gew.-% eines oder mehrerer Copolymere umfassend mindestens eine Struktureinheit der Formel (I) und mindestens eine Struktureinheit der Formel (II) und mindestens eine Struktureinheit der Formel (III) 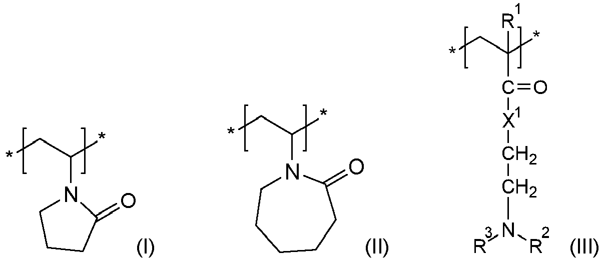 worin
      R¹ ein Wasserstoffatom oder eine Methylgruppe bedeutet,
      X¹ ein Sauerstoffatom oder eine Gruppe NH bedeutet,
      R² und R³ unabhängig voneinander für eine (C₁ bis C₄)-Alkylgruppe stehen
      und
   (c) 0,15 bis 1,5 Gew.-% eines oder mehrerer Copolymere umfassend mindestens eine Struktureinheit der Formel (I) und mindestens eine Struktureinheit der Formel (IV) 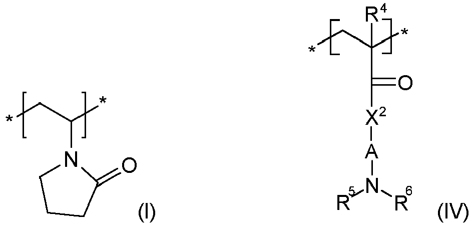 worin
      R⁴ ein Wasserstoffatom oder eine Methylgruppe bedeutet,
      X² ein Sauerstoffatom oder eine Gruppe NH bedeutet,
      A eine (C₂ bis C₃)-Alkylengruppe (insbesondere Ethan-1,2-diyl oder Propan-1,3-diyl),
      R⁵ und R⁶ unabhängig voneinander für eine (C₁ bis C₄)-Alkylgruppe stehen
      und
   (d) mindestens ein kationisches Tensid, ausgewählt unter N-(C₁₂ bis C₂₂)Alkyl-N,N,N-trimethylammonium-Verbindungen
      und
   (e) mindestens ein kationisches Tensid gemäß Formel (VI), 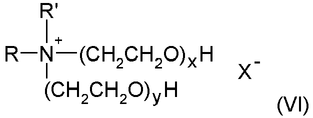 worin
      x und y stehen unabhängig voneinander für eine ganze Zahl größer 0,
      R steht für eine (C₈ bis C₂₀)-Alkylgruppe oder eine (C₈ bis C₂₀)-Alkenylgruppe,
      R' steht für eine Gruppe *-(CH₂CH₂O)_{z}H worin z eine ganze Zahl größer 0 bedeutet, eine (C₈ bis C₂₀)-Alkylgruppe oder eine (C₈ bis C₂₀)-Alkenylgruppe,
      X⁻ steht für ein physiologisch verträgliches Anion.
(K): Mittel zur Behandlung keratinhaltiger Fasern, insbesondere menschlichem Haar, enthaltend in einem kosmetischen Träger
   (a) 0,1 bis 3,0 Gew.-% einer oder mehrerer kationischer Celluloseverbindungen der INCI-Bezeichnung Polyquaternium-4
      und
   (b) 0,1 bis 10,0 Gew.-% eines oder mehrerer Copolymere der INCI-Bezeichnung Vinylcaprolactam/VP/Dimethylaminoethyl-methacrylate Copolymer
      und
   (c) 0,01 bis 5,0 Gew.-% eines oder mehrerer Copolymere ausgewählt aus mindestens einem Copolymer der Gruppe, die gebildet wird aus Copolymeren der INCI-Bezeichnung Polyquaternium-11 und Copolymeren der INCI-Bezeichnung VP/DMAPA Acrylates Copolymer
      und
   (d) mindestens ein kationisches Tensid, ausgewählt unter N-(C₁₂ bis C₂₂)Alkyl-N,N,N-trimethylammonium-Verbindungen
      und
   (e) mindestens ein kationisches Tensid gemäß Formel (VI), 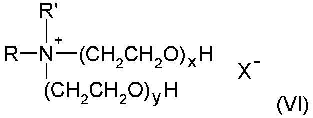 worin
      x und y stehen unabhängig voneinander für eine ganze Zahl größer 0,
      R steht für eine (C₈ bis C₂₀)-Alkylgruppe oder eine (C₈ bis C₂₀)-Alkenylgruppe,
      R' steht für eine Gruppe *-(CH₂CH₂O)_{z}H worin z eine ganze Zahl größer 0 bedeutet, eine (C₈ bis C₂₀)-Alkylgruppe oder eine (C₈ bis C₂₀)-Alkenylgruppe,
      X⁻ steht für ein physiologisch verträgliches Anion.
(L): Mittel zur Behandlung keratinhaltiger Fasern, insbesondere menschlichem Haar, enthaltend in einem kosmetischen Träger
   (a) 0,8 bis 1,2 Gew.-% einer oder mehrerer kationischer Celluloseverbindungen der INCI-Bezeichnung Polyquaternium-4
      und
   (b) 0,5 bis 5,0 Gew.-% eines oder mehrerer Copolymere der INCI-Bezeichnung Vinylcaprolactam/VP/Dimethylaminoethylmethacrylate Copolymer und
   (c) 0,15 bis 1,5 Gew.-% eines oder mehrerer Copolymere ausgewählt aus mindestens einem Copolymer der Gruppe, die gebildet wird aus Copolymeren der INCI-Bezeichnung Polyquaternium-11 und Copolymeren der INCI-Bezeichnung VP/DMAPA Acrylates Copolymer und
   (d) mindestens ein kationisches Tensid, ausgewählt unter N-(C₁₂ bis C₂₂)Alkyl-N,N,N-trimethylammonium-Verbindungen
      und
   (e) Quaternium-52.

Die erfindungsgemäßen Mittel können optional zusätzlich mindestens ein weiteres festigendes Polymer enthalten, das von den Polymeren (a), (b) und (c) verschieden ist, unter der Voraussetzung, dass deren Einsatz den Effekt der erfindungsgemäßen Polymerkombination nicht aufhebt. Die optional zugesetzten festigenden Polymere sind bevorzugterweise kationisch und/oder nichtionisch.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curlretention - Test angewendet.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Geeignete festigende kationische Polymere sind beispielsweise als
- Copolymere aus Methacryloylaminopropyllauryldimethylammonium chlorid mit N-Vinylpyrrolidon und Dimethylaminopropylmethacrylamid mit der INCI-Bezeichnung Polyquaternium-55 unter den Handelsnamen Styleze^{®} W-10, Styleze^{®} W-20 (Firma ISP),
- Copolymere aus Methacryloylaminopropyllauryldimethylammonium chlorid mit N-Vinylpyrrolidon, N-Vinylcaprolactam und Dimethylaminopropylmethacrylamid mit der INCI-Bezeichnung Polyquaternium-69 unter den Handelsnamen Aquastyle^{®} 300 (Firma ISP),
- Copolymere aus N-Methylvinylimidazol und Vinylpyrrolidon mit der INCI-Bezeichnung Polyquaternium-16 unter den Handelsnamen Luviquat^{®} Style, Luviquat^{®} FC 370, Luviquat^{®} FC 550, Luviquat^{®} FC 905 und Luviquat^{®} HM 552, oder als Polyquaternium-44 unter dem Handelsnamen Luviquat^{®} UltraCare von der Firma BASF SE,
- Copolymere aus N-Methylvinylimidazol und Vinylpyrrolidon und Vinylcaprolactam-Copolymere mit der INCI-Bezeichnung Polyquarternium-46 und unter dem Handelsnamen Luviquat^{®} Hold von der Firma BASF SE,
- Copolymere aus N-Methylvinylimidazol und Vinylpyrrolidon und Vinylimidazol und Methacrylamid mit der INCI-Bezeichnung Polyquaternium-68 und unter dem Handelsnamen Luviquat^{®} Supreme von der Firma BASF SE,
im Handel erhältlich.

Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Geeignete festigende nichtionische Polymere sind beispielsweise
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und/oder an gehärtetes Rizinusöl enthalten. Die Anlagerungsprodukte von 5 bis 60 Mol Ethylenoxid an Rizinusöl gehärtetes Rizinusöl sind erfindungsgemäß am bevorzugtesten.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.
Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole verstanden.
Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning^{®} 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.
Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein. Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning^{®} 939 oder als Handelsprodukt Dow Corning^{®} 949 im Gemisch mit einem kationischen und eine nichtionischen Tensid erhältlich.
Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a. Vitamin B₁ (Thiamin), Vitamin B₂ (Riboflavin), Vitamin B₃ (Nicotinsäure und/oder Nicotinsäureamid (Niacinamid)), Vitamin B₅ (Pantothensäure, Panthenol und Pantolacton), Vitamin B₆ (Pyridoxin sowie Pyridoxamin und Pyridoxal), Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.
Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden.
Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.
Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.
Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.
Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).
Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 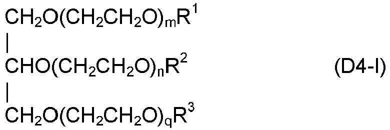 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.
Die Farbstoffe, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.
Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel. Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäßen Mittel als Pumpspray, Aerosolspray, Pumpschaum oder Aerosolschaum.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäßen Mittel als Aerosolhaarschaum oder Aerosolhaarspray konfektioniert. Das erfindungsgemäße Mittel enthält daher bevorzugt zusätzlich mindestens ein Treibmittel.

Erfindungsgemäß geeignete Treibmittel sind beispielsweise ausgewählt aus N₂, N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus N₂O, Dimethylether, CO₂, Luft, Alkanen mit 3 oder 4 Kohlenstoffatomen (wie Propan, n-Butan, iso-Butan) sowie Mischungen daraus.

Ein zweiter Gegenstand der Erfindung ist die Verwendung der erfindungsgemäßen Mittel zur temporären Verformung von Haaren, insbesondere zur Volumengebung.

Die erfindungsgemäßen Mittel und Produkte, die diese Mittel enthalten, insbesondere Aerosolhaarschäume bzw. Aerosolhaarsprays, zeichnen sich insbesondere dadurch aus, dass sie behandeltem Haar ein sehr starkes, haltbares Volumen verleihen, obgleich das Haar flexibel bleibt. Wird das Mittel als Haarschaum konfektioniert, bildet sich ein stabiler, feinporiger und cremiger Schaum, der sich gleichmäßig und ohne zu tropfen auf dem Haar verteilen lässt. Es bilden sich keine sichtbaren Rückstände auf dem Haar.

Ein dritter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand zu einem Schaum verschäumt wird und der resultierende Schaum auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen *(vide supra*). Ein vierter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Spray auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen *(vide supra*).

Die nachfolgenden Beispiele sollen den Gegenstand der vorliegenden Erfindung erläutern ohne ihn in irgendeiner Weise zu beschränken.

### Beispiele

Alle Mengenangaben sind, falls nicht anders gekennzeichnet - als Gewichtsprozent angegeben. Folgende Rohstoffe wurden zur Bereitstellung der nachfolgenden Rezeptur eingesetzt:

**Tabelle 1: Rezeptur**

| **Rohstoff** | **E1** |
|---|---|
| Milchsäure | 0,04 |
| Natrium Benzoat | 0,3 |
| D-Panthenol | 0,2 |
| Dehyquart A CA ¹ | 1,0 |
| Dehyquart SP ² | 0,5 |
| PEG-40 Hydrogenated Castor Oil | 0,2 |
| Celquat L-200 ³ | 1,0 |
| Gafquat 755N-PW ⁴ | 6,0 |
| Advantage S ⁵ | 2,5 |
| Wasser, vollentsalzt | ad 100 |
| Propan/Butan | 8,0 |

| | |
|---|---|
| ¹ Trimethylhexadecylammoniumchlorid (ca. 24 -26 % Aktivsubstanz; INCI-Bezeichnung: Aqua (Water), Cetrimonium Chloride) (Cognis) ² Tris-(oligooxyethyl)alkylammoniumphosphat (ca. 49-51% Aktivsubstanz in Wasser; INCI-Bezeichnung: Aqua (Water), Quaternium-52, Propylene Glycol) (Cognis) ³ quaterniertes Cellulose-Derivat (INCI-Bezeichnung: Polyquaternium-4) (National Starch) ⁴ Dimethylaminoethylmethacrylat-Vinylpyrrolidon-Copolymer, quaterniert mit Diethylsulfat (ca. 19% Festkörper in Wasser; INCI-Bezeichnung: Polyquaternium-11; ISP) ⁵ INCI-Bezeichnung: Vinylcaprolactam/VP/Dimethyl-aminoethylmethacrylate Copolymer (in Pulverform; ISP) | |

Die Rezepturbestandteile wurden jeweils in ein Aerosolbehältnis gefüllt, das folgende technische Parameter erfüllt: Aluminium Vorratsbehältnis mit Ventil Produkt 522983 PV10697 der Firma Pecision (Deutsche Präzisions-Ventil GmbH).

Die Rezepturen wurden jeweils als Haarschaum aus dem Aerosolbehälter ausgebracht und auf dem Haar von Probanden verteilt. Die Rezeptur verlieh der Frisur der Probanden (auch nachprüfbar durch Durchführung eines High Humidity Curl Retention Tests) einen hohen Halt und Volumen. Ferner entstanden auch Stunden nach der Applikation keine Filmplaken. Selbst nach dem Ausbürsten wurden keine Filmplaken festgestellt.

## Patentansprüche

1. Mittel zur Behandlung keratinhaltiger Fasern, insbesondere menschlichem Haar, enthaltend in einem kosmetischen Träger
(a) mindestens eine kationische Celluloseverbindung
und
(b) mindestens ein Copolymer umfassend mindestens eine Struktureinheit der Formel (I) und mindestens eine Struktureinheit der Formel (II) und mindestens eine Struktureinheit der Formel (III) 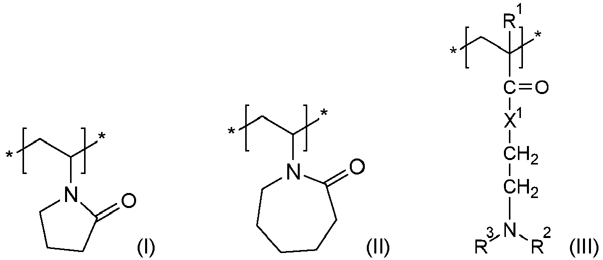 worin
R¹ ein Wasserstoffatom oder eine Methylgruppe bedeutet,
X¹ ein Sauerstoffatom oder eine Gruppe NH bedeutet,
R² und R³ unabhängig voneinander für eine (C₁ bis C₄)-Alkylgruppe stehen
und
(c) mindestens ein Copolymer umfassend mindestens eine Struktureinheit der Formel (I) und mindestens eine Struktureinheit der Formel (IV) 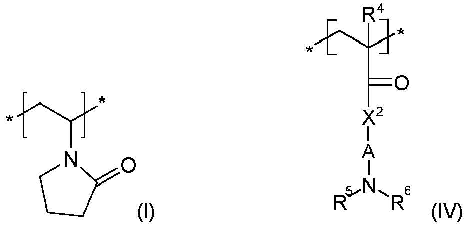 worin
R⁴ ein Wasserstoffatom oder eine Methylgruppe bedeutet,
X² ein Sauerstoffatom oder eine Gruppe NH bedeutet,
A eine (C₂ bis C₃)-Alkylengruppe (insbesondere Ethan-1,2-diyl oder Propan-1,3-diyl),
R⁵ und R⁶ unabhängig voneinander für eine (C₁ bis C₄)-Alkylgruppe stehen.

2. Mittel nach Anspruch 1, **dadurch gekennzeichnet, dass** die kationische Celluloseverbindung in mindestens einer Seitenkette mehr als eine permanente kationische Ladung trägt.

3. Mittel nach einem der Ansprüche 1 oder 2, **dadurch gekennzeichnet, dass** die kationischen Celluloseverbindungen (a) in einer Menge von 0,1 Gew.-% bis 3,0 Gew.-%, bevorzugt von 0,4 Gew.-% bis 1,8 Gew.-%, besonders bevorzugt von 0,8 Gew.-% bis 1,2 Gew.-%, jeweils bezogen auf das Gewicht des erfindungsgemäßen Mittels, enthalten sind.

4. Mittel nach einem der Ansprüche 1 bis 3, **dadurch gekennzeichnet, dass** als Copolymer (b) mindestens ein Terpolymer enthalten ist, das erhalten wird aus N-Vinylpyrrolidon, N-Vinylcaprolactam und N,N-Dimethylaminoethylmethacrylat.

5. Mittel nach einem der Ansprüche 1 bis 4, **dadurch gekennzeichnet, dass** die Copolymere (b) in einer Menge von 0,1 Gew.-% bis 10,0 Gew.-%, bevorzugt von 0,5 Gew.-% bis 8,0 Gew.-%, besonders bevorzugt von 0,5 Gew.-% bis 5,0 Gew.-%, jeweils bezogen auf das Gewicht des erfindungsgemäßen Mittels, enthalten sind.

6. Mittel nach einem der Ansprüche 1 bis 5, **dadurch gekennzeichnet, dass** gemäß Formel (IV)
R⁴ eine Methylgruppe bedeutet,
X² ein Sauerstoffatom oder eine Gruppe NH bedeutet,
A für Ethan-1,2-diyl oder Propan-1,3-diyl steht und
R⁵ und R⁶ unabhängig voneinander für eine (C₁ bis C₄)-Alkylgruppe (insbesondere Methyl) stehen,
mit der Maßgabe, dass
wenn X² für ein Sauerstoffatom steht, bedeutet A Ethan-1,2-diyl,
wenn X² für eine Gruppe NH steht, bedeutet A Propan-1,3-diyl.

7. Mittel nach Anspruch 6, **dadurch gekennzeichnet, dass** X² gemäß Formel (IV) für ein Sauerstoffatom steht und das Copolymer (c) zusätzlich mindestens eine Struktureinheit der Formel (V) umfasst 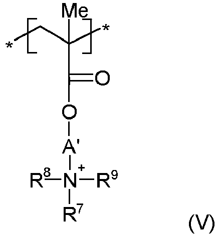 in der
R⁸ und R⁹ unabhängig voneinander für eine (C₁ bis C₆)-Alkylgruppe (insbesondere für Methyl) stehen, A' für Ethan-1,2-diyl oder Ethan-1,3-diyl (insbesondere Ethan-1,2-diyl) und R⁷ für eine (C₁ bis C₄)-Alkylgruppe (insbesondere für Ethyl) steht.

8. Mittel nach einem der Ansprüche 1 bis 7, **dadurch gekennzeichnet, dass** die Copolymere (c) in einer Menge von 0,01 Gew.-% bis 5,0 Gew.-%, bevorzugt von 0,1 Gew.-% bis 2,0 Gew.-%, besonders bevorzugt von 0,15 Gew.-% bis 1,5 Gew.-%, jeweils bezogen auf das Gewicht des erfindungsgemäßen Mittels, enthalten sind.

9. Mittel nach einem der Ansprüche 1 bis 8, **dadurch gekennzeichnet, dass** zusätzlich mindestens ein kationisches Tensid enthalten ist.

10. Mittel nach einem der Ansprüche 1 bis 9, **dadurch gekennzeichnet, dass** mindestens ein Treibmittel enthalten ist.

11. Verwendung eines Mittels gemäß einem der Ansprüche 1 bis 10 zur temporären Verformung von Haaren, insbesondere zur Volumengebung.

12. Verfahren zur Behandlung keratinhaltiger Fasern, insbesondere menschlicher Haare, worin unter Einsatz einer Abgabevorrichtung ein Mittel gemäß einem der Ansprüche 1 bis 10 zu einem Schaum verschäumt wird und der resultierende Schaum auf die keratinhaltigen Fasern appliziert wird.

13. Verfahren zur Behandlung keratinhaltiger Fasern, insbesondere menschlicher Haare, worin unter Einsatz einer Abgabevorrichtung ein Mittel gemäß einem der Ansprüche 1 bis 10 als Spray auf die keratinhaltigen Fasern appliziert wird.

## Claims

1. An agent for treating keratin-containing fibers, in particular human hair, containing, in a cosmetic carrier,
(a) at least one cationic cellulose compound and
(b) at least one copolymer comprising at least one structural unit of formula (I) and at least one structural unit of formula (II) and at least one structural unit of formula (III) 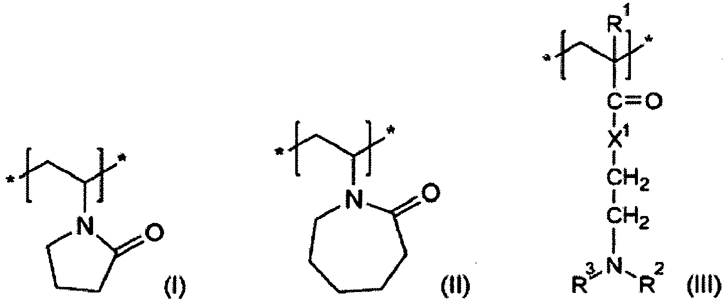 where
R¹ denotes a hydrogen atom or a methyl group,
X¹ denotes an oxygen atom or an NH group,
R² and R³ represent, independently of one another, a (C₁ to C₄) alkyl group,
and
(c) at least one copolymer comprising at least one structural unit of formula (I) and at least one structural unit of formula (IV) 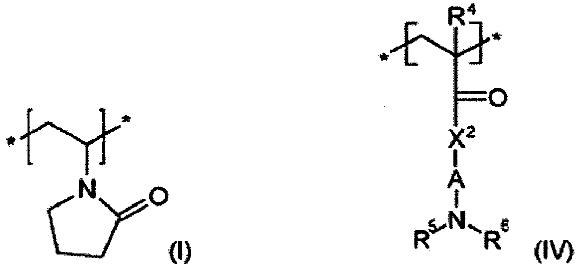 where
R⁴ denotes a hydrogen atom or a methyl group,
X² denotes an oxygen atom or an NH group,
A represents a (C₂ to C₃) alkylene group (in particular ethane-1,2-diyl or propane-1,3-diyl), and
R⁵ und R⁶ represent, independently of one another, a (C₁ to C₄) alkyl group.

2. The agent according to claim 1, **characterized in that** the cationic cellulose compound has more than one permanent cationic charge in at least one side chain.

3. The agent according to claim 1 or 2, **characterized in that** the cationic cellulose compounds (a) are contained in an amount of from 0.1 wt.% to 3.0 wt.%, preferably from 0.4 wt.% to 1.8 wt.%, particularly preferably from 0.8 wt.% to 1.2 wt.%, in each case based on the weight of the agent according to the invention.

4. The agent according to one of claims 1 to 3, **characterized in that** at least one terpolymer is contained as the copolymer (b) and is obtained from N-vinylpyrrolidone, N-vinylcaprolactam and N,N-dimethylaminoethyl methacrylate.

5. The agent according to one of claims 1 to 4, **characterized in that** the copolymers (b) are contained in an amount of from 0.1 wt.% to 10.0 wt.%, preferably from 0.5 wt.% to 8.0 wt.%, particularly preferably from 0.5 wt.% to 5.0 wt.%, in each case based on the weight of the agent according to the invention.

6. The agent according to one of claims 1 to 5, **characterized in that**, according to formula (IV), R⁴ denotes a methyl group,
X² denotes an oxygen atom or an NH group,
A represents ethane-1,2-diyl or propane-1,3-diyl, and
R⁵ and R⁶ represent, independently of one another, a (C₁ to C₄) alkyl group (in particular methyl),
with the proviso that,
if X² represents an oxygen atom, A denotes ethane-1,2-diyl,
if X² represents an NH group, A denotes propane-1,3-diyl.

7. The agent according to claim 6, **characterized in that**, according to formula (IV), X² represents an oxygen atom, and the copolymer (c) additionally comprises at least one structural unit of formula (V) 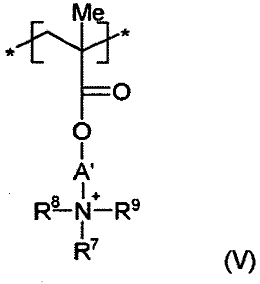 where R⁸ and R⁹ represent, independently of one another, a (C₁ to C₆) alkyl group (in particular methyl), A' represents ethane-1,2-diyl or ethane-1,3-diyl (in particular ethane-1,2-diyl), and R⁷ represents a (C₁ to C₄) alkyl group (in particular ethyl).

8. The agent according to one of claims 1 to 7, **characterized in that** the copolymers (c) are contained in an amount of from 0.01 wt.% to 5.0 wt.%, preferably from 0.1 wt.% to 2.0 wt.%, particularly preferably from 0.15 wt.% to 1.5 wt.%, in each case based on the weight of the agent according to the invention.

9. The agent according to one of claims 1 to 8, **characterized in that** at least one cationic surfactant is additionally contained.

10. The agent according to one of claims 1 to 9, **characterized in that** at least one propellant is contained.

11. The use of an agent according to one of claims 1 to 10 for temporarily shaping hair, in particular for giving volume.

12. A method for treating keratin-containing fibers, in particular human hair, wherein, using a dispensing device, an agent according to one of claims 1 to 10 is foamed to form a mousse and the resultant mousse is applied to the keratin-containing fibers.

13. A method for treating keratin-containing fibers, in particular human hair, wherein, using a dispensing device, an agent according to one of claims 1 to 10 is applied to the keratin-containing fibers as a spray.

## Revendications

1. Produit de traitement de fibres de kératine, en particulier de cheveux humains, contenant dans un vecteur cosmétique :
(a) au moins un composé cellulosique cationique, et
(b) au moins un copolymère contenant au moins une unité structurelle de formule (I) et au moins une unité structurelle de formule (II) et au moins une unité structurelle de formule (III) : 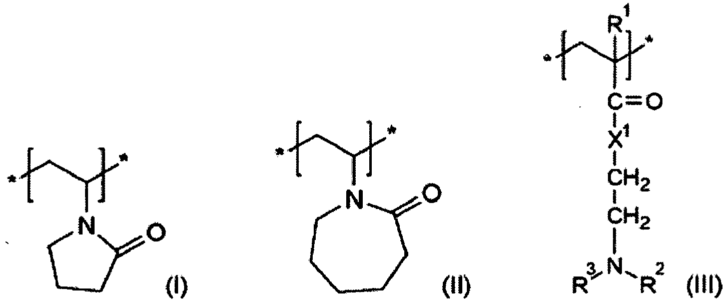 où
R¹ désigne un atome d'hydrogène ou un groupe méthyle
X¹ désigne un atome d'oxygène ou un groupe NH,
R² et R³ représentent, indépendamment l'un de l'autre, un groupe C₁₋₄-alkyle, et
(c) au moins un copolymère contenant au moins une unité structurelle de formule (I) et au moins une unité structurelle de formule (IV) : 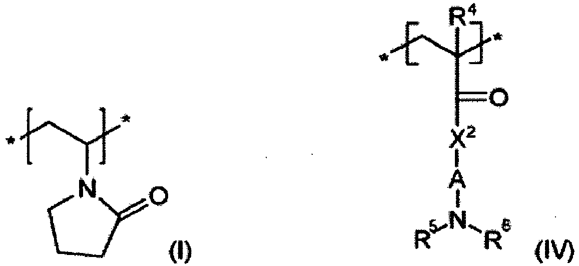 où :
R⁴ désigne un atome d'hydrogène ou un groupe méthyle
X² désigne un atome d'oxygène ou un groupe NH,
A représente un groupe C₂₋₃-alkylène (en particulier éthan-1,2-diyle ou propan-1,3-diyle),
R⁵ et R⁶ représentent, indépendamment l'un de l'autre, un groupe C₁₋₄-alkyle.

2. Produit selon la revendication 1, **caractérisé en ce que** le composé cellulosique cationique porte, dans au moins une chaîne latérale, plusieurs charges cationiques permanentes.

3. Produit selon une des revendications 1 ou 2, **caractérisé en ce que** les composés cellulosiques cationiques (a) sont contenus à hauteur de 0,1 % à 3,0 % en poids, de préférence de 0,4 % à 1,8 % en poids, particulièrement préférentiellement de 0,8 % à 1,2 % en poids, rapportés respectivement au poids du produit prêt à l'emploi.

4. Produit selon une des revendications 1 à 3, **caractérisé en ce qu'**il contient comme copolymère (b) au moins un terpolymère obtenu à partir de la N-vinylpyrrolidone, du N-vinylcaprolactame et du méthacrylate de N,N-diméthylaminoéthyle.

5. Produit selon une des revendications 1 à 4, **caractérisé en ce que** les copolymères (b) sont contenus à hauteur de 0,1 % à 10,0 % en poids, de préférence de 0,5 % à 8,0 % en poids, particulièrement préférentiellement de 0,5 % à 5,0 % en poids, rapportés respectivement au poids du produit prêt à l'emploi.

6. Produit selon une des revendications 1 à 5, **caractérisé en ce que** selon la formule (IV) :
R⁴ représente un groupe méthyle
X² désigne un atome d'oxygène ou un groupe NH,
A représente éthan-1,2-diyle ou propan-1,3-diyle, et
R⁵ et R⁶ représentent, indépendamment l'un de l'autre, un groupe C₁₋₄-alkyle (en particulier méthyle),
à condition que :
si X² désigne un atome d'oxygène, A signifie éthan-1,2-diyle,
si X² désigne un groupe NH, A signifie propan-1,3-diyle.

7. Produit selon la revendication 6, **caractérisé en ce que** X² selon la formule (IV) représente un atome d'oxygène, et **en ce que** le copolymère (c) comprend en plus une unité structurelle de formule (V) : 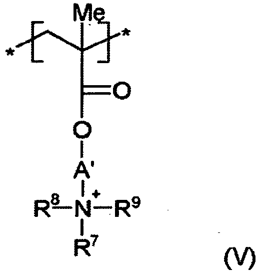 où :
R⁸ et R⁹ représentent, indépendamment l'un de l'autre, un groupe C₁₋₆-alkyle (en particulier méthyle), A' représente éthan-1,2-diyle ou propan-1,3-diyle (en particulier éthan-1,2-diyle) et R⁷ représente un groupe C₁₋₄-alkyle (en particulier éthyle).

8. Produit selon une des revendications 1 à 7, **caractérisé en ce que** les copolymères (c) sont contenus à hauteur de 0,1 % à 5,0 % en poids, de préférence de 0,1 % à 2,0 % en poids, particulièrement préférentiellement de 0,15 % à 1,5 % en poids, rapportés respectivement au poids du produit prêt à l'emploi.

9. Produit selon une des revendications 1 à 8, **caractérisé en ce qu'**il contient en plus au moins un tensioactif cationique.

10. Produit selon une des revendications 1 à 9, **caractérisé en ce qu'**il contient en plus au moins un agent propulseur.

11. Utilisation d'un produit selon une des revendications 1 à 10 pour déformer temporairement les cheveux, en particulier pour leur donner du volume.

12. Procédé de traitement de fibres de kératine, en particulier de cheveux humains, où en utilisant un dispositif de délivrance, on fait mousser un produit selon une des revendications 1 à 10 et on applique la mousse obtenue sur les fibres de kératine.

13. Procédé de traitement de fibres de kératine, en particulier de cheveux humains, où en utilisant un dispositif de délivrance, on applique sur les fibres de kératine un produit selon une des revendications 1 à 10 sous forme de spray.
